# EUROPEAN PATENT SPECIFICATION

(11) **EP 3 001 673 B1**
(45) Date of publication and mention of the grant of the patent: **28.02.2018**
(21) Application number: 14801714.8
(22) Date of filing: 28.03.2014
(51) Int. Cl.: H04N 5/225, A61B 1/04, G02B 23/24, H01L 27/14, H04N 5/335, H01L 27/146, A61B 1/05, G02B 27/62, H04N 7/18

(54) **IMAGE PICKUP APPARATUS, IMAGE PICKUP APPARATUS MANUFACTURING METHOD, AND ENDOSCOPE SYSTEM**
BILDAUFNAHMEVORRICHTUNG, HERSTELLUNGSVERFAHREN FÜR BILDAUFNAHMEVORRICHTUNG UND ENDOSKOPSYSTEM
APPAREIL DE PRISE D'IMAGE, PROCÉDÉ DE FABRICATION D'APPAREIL DE PRISE D'IMAGE ET SYSTÈME ENDOSCOPIQUE

(30) Priority: 22.05.2013 JP 2013108138
(43) Date of publication of application: 30.03.2016
(73) Proprietor: Olympus Corporation, Tokyo 192-8507 (JP)
(72) Inventor: FUJIMORI Noriyuki, Tokyo 192-8507 (JP)
(74) Representative: Winter, Brandl, Fürniss, Hübner, Röss, Kaiser, Polte - Partnerschaft mbB
(86) International application number: PCT/JP2014/059258
(87) International publication number: WO 2014/188788

(56) References cited:
- WO-A1-2007/034929
- JP-A- S5 944 006
- JP-A- H04 235 475
- JP-A- H04 235 475
- US-A1- 2001 024 848
- US-A1- 2011 224 487
- US-A1- 2012 201 433
- US-B1- 6 670 205

## Description

### Technical Field

The present invention relates to an image pickup apparatus that includes an image pickup chip in which an optical member is bonded on a light-receiving portion, a method for manufacturing the image pickup apparatus, and an endoscope system that includes the image pickup apparatus.

### Background Art

An image pickup apparatus including an image pickup chip is, for example, arranged at a distal end portion of an electronic endoscope and used. An important task with regard to electronic endoscopes is to reduce a diameter of the distal end portion thereof, and consequently there is a demand to miniaturize the image pickup apparatus.

First, for comparison purposes, a wafer-level-packaging-type image pickup apparatus will be briefly described. A wafer-level-packaging-type image pickup apparatus is manufactured by cutting into individual pieces a bonded wafer in which an image pickup wafer that includes a plurality of image pickup chips and a glass wafer are bonded. Therefore, an entire area of a first main surface on which a light-receiving portion of an image pickup chip is formed is covered by a cover glass. It is necessary to form an electrode pad for sending and receiving signals to and from the light-receiving portion on a second main surface (rear surface) via through-wiring. However, a through-wiring formation process is a complex process that includes through-hole formation, insulating layer formation, and conductor layer formation and the like.

Japanese Patent Application Laid-Open Publication No. 2008-118568 discloses an image pickup apparatus in which a light-receiving portion and an electrode pad are arranged on a first main surface. Unlike a wafer-level-packaging-type image pickup apparatus, it is not necessary to form through-wiring in the aforementioned image pickup apparatus, and it is thus easy to produce the image pickup apparatus. However, a cover glass for protecting the light-receiving portion of an image pickup chip is positioned so as not to cover the electrode pad, and it is necessary to bond the cover glass. However, in a case where each side of the image pickup chip is a small size of a number of millimeters or less, it is not easy to accurately position the cover glass so as to cover the light-receiving portion and not cover the electrode pad and then bond the cover glass.

In a case where positioning accuracy is inadequate, there is a problem that if a side face of the cover glass is too near the light-receiving portion, an optical flare will be generated as the result of light that is incident from an image pickup optical system being reflected by the side face of the cover glass to form reflected light that thereafter arrives at the light-receiving portion.

Therefore, while it is also conceivable to bond a cover glass that has a plan-view dimension that is significantly larger than the image pickup chip taking into account the fact that the positioning accuracy is low, in such a case there will be a problem that an external diameter of the image pickup apparatus will increase.

Consequently, there is a need for an image pickup apparatus that is easily manufactured in which a light-receiving portion and an electrode pad are arranged on a main surface and in which the light-receiving portion is covered by a cover glass, a method for manufacturing the image pickup apparatus, and an endoscope system that includes the image pickup apparatus.

WO2007/034929 A1 discloses an imaging apparatus and its manufacturing method using alignment marks to position a glass cover plate on a CCD wafer.

US2001/0024848 A1 discloses use of a light shielding metal film comprising alignment marks and a moisture-resistant wall surrounding a light receiving portion.

JP04235475 discloses alignment of a transparent based board and a solid-state image pickup element using alignment marks formed on a bonding surface of the transparent base board. US2012/0201433 A1 discloses an endoscope system in which corner portions of the endoscopic image are masked.

### Summary of Invention

### Technical Problem

An object of the present invention is to provide an image pickup apparatus that is easily manufactured in which a light-receiving portion and an electrode pad are arranged on a main surface and in which the light-receiving portion is covered by a cover glass, a method for manufacturing the image pickup apparatus, and an endoscope system that includes the image pickup apparatus.

### Solution to Problem

An image pickup apparatus according to the present invention is defined by claim 1 and includes: a rectangular parallelepiped image pickup chip made of a semiconducting material and in which a plurality of function portion patterns including a light-receiving portion are formed on a first main surface; and an optical member in which an alignment mark is formed at each of at least two places that are in a predetermined positional relationship with a function portion pattern, and which is made of a transparent material that is bonded via an adhesive layer so as to cover the light-receiving portion.

A method for manufacturing an image pickup apparatus according to the present invention is defined by claim 11 and includes: a process of fabricating an image pickup chip wafer made of a semiconducting material in which a plurality of image pickup chip patterns made of a plurality of function portion patterns that each include a light-receiving portion are formed on a first main surface; a process of fabricating a plurality of rectangular parallelepiped image pickup chips by cutting the image pickup chip wafer into individual pieces; a process of forming, on an optical member wafer, a plurality of alignment mark sets, in each of which alignment marks are respectively formed at positions that are in a predetermined positional relationship with at least two places of the function portion pattern of the image pickup chip pattern; a process of fabricating a plurality of optical members that are rectangular in a planar view and on each of which one of the alignment marks set is formed, by cutting the optical member wafer into individual pieces; and a process of bonding the image pickup chip and the optical member via an adhesive layer while aligning an alignment mark of the optical member and the function portion pattern that is in a predetermined positional relationship with the alignment mark.

An endoscope system according to the present invention is defined by claim 14 and includes: an endoscope including a rectangular parallelepiped image pickup chip made of a semiconducting material and in which a plurality of function portion patterns including a light-receiving portion are formed on a first main surface, and an optical member in which an alignment mark is formed at each of at least two places that are in a predetermined positional relationship with a function portion pattern and which is made of a transparent material that is bonded via an adhesive layer so as to cover the light-receiving portion, wherein: the alignment mark in plurality that are formed by a metal layer are formed on a bonding surface of the optical member, the optical member is a cover glass, a filter, a prism or a member having a lens function, and an image pickup apparatus in which the optical member is bonded so that the alignment mark covers a corner portion of the light-receiving portion is arranged at a distal end portion of an insertion portion; and a processor having a processing portion that processes a rectangular image pickup image that the image pickup apparatus outputs, and outputs an endoscopic image that masks and does not display the corner portion of the image pickup image.

### Advantageous Effects of Invention

According to the present invention, an image pickup apparatus that is easily manufactured in which a light-receiving portion and an electrode pad are arranged on a main surface and in which the light-receiving portion is covered by a cover glass, a method for manufacturing the image pickup apparatus, and an endoscope system that includes the image pickup apparatus can be provided.

### Brief Description of the Drawings

Fig. 1 is a perspective view of an image pickup apparatus of a first embodiment;
Fig. 2 is an exploded view for describing a method for manufacturing the image pickup apparatus of the first embodiment;
Fig. 3 is a flowchart for describing the method for manufacturing the image pickup apparatus of the first embodiment;
Fig. 4A is a plan view of an image pickup wafer of the image pickup apparatus of the first embodiment;
Fig. 4B is a plan view of an image pickup chip of the image pickup apparatus of the first embodiment;
Fig. 5A is a plan view of a glass wafer of the image pickup apparatus of the first embodiment;
Fig. 5B is a plan view of a cover glass of the image pickup apparatus of the first embodiment;
Fig. 6 is a plan view for describing an alignment pattern of the image pickup apparatus of the first embodiment;
Fig. 7 is a plan view for describing an alignment pattern of an image pickup apparatus of Modification 1 of the first embodiment;
Fig. 8 is a plan view of an image pickup apparatus of Modification 2 of the first embodiment;
Fig. 9 is a plan view of an image pickup apparatus of Modification 3 of the first embodiment;
Fig. 10 is a cross-sectional schematic diagram of an image pickup apparatus of Modification 4 of the first embodiment;
Fig. 11 is a plan view of an image pickup apparatus of Modification 5 of the first embodiment;
Fig. 12 is a plan view of an image pickup apparatus of Modification 6 of the first embodiment;
Fig. 13 is an exploded view for describing an image pickup apparatus of Modification 7 of the first embodiment;
Fig. 14 is a configuration diagram of an endoscope system of a second embodiment;
Fig. 15A illustrates an endoscopic image prior to processing by the endoscope system of the second embodiment; and
Fig. 15B illustrates an endoscopic image after processing by the endoscope system of the second embodiment.

### Best Mode for Carrying Out the Invention

### <First Embodiment>

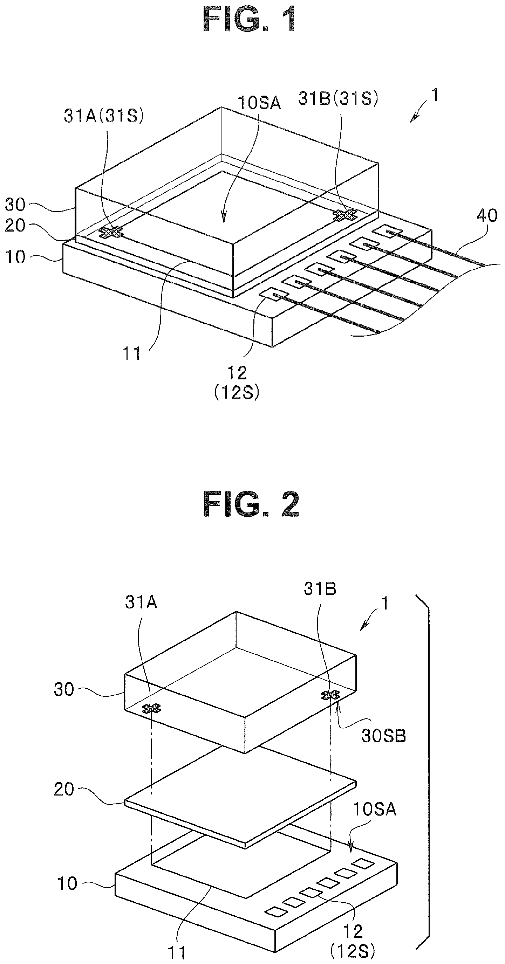

As shown in Fig. 1, an image pickup apparatus 1 of the present embodiment includes an image pickup chip 10, and a cover glass 30 that is an optical member made of a transparent material that is bonded via an adhesive layer 20 to the image pickup chip 10. Note that, although signal wires 40 are also illustrated in Fig. 1, in the following description the flexible long signal wires 40 are not taken as constituent elements of the image pickup apparatus 1.

The rectangular parallelepiped image pickup chip 10 is an image pickup device such as a CMOS image sensor made of a semiconducting material such as silicon, in which a light-receiving portion 11 is formed on a first main surface 10SA. An electrode pattern (hereunder, also referred to as a "electrode pad") 12 that is connected by wiring (not shown) for sending and receiving signals to and from the light-receiving portion 11 is also arranged on the first main surface 10SA. A plurality of the electrode pads 12 to which the signal wires 40 are bonded, respectively, are provided in a row along an edge of the image pickup chip 10. Hereunder, a pattern that is formed on the first main surface 10SA for an image pickup function is referred to as a function portion pattern. That is, in the image pickup chip 10, the light-receiving portion 11, the wiring and the electrode pattern 12 are function portion patterns.

The cover glass 30 is positioned so as to cover the light-receiving portion 11 and not cover an electrode group 12S that includes a plurality of the electrode pads 12, and is bonded to the image pickup chip 10.

With regard to outer dimensions (plan-view dimensions) of the image pickup chip 10, each side thereof is an extremely small size of a number of millimeters, for example, 1 mm. Consequently, as described above, it is not easy to align the image pickup chip 10 and the cover glass 30.

However, in the image pickup apparatus 1, on a bonding surface (second main surface) 30SB of the cover glass 30, alignment marks 31A and 31B (alignment mark set 31S) are respectively formed at two places that are in a predetermined positional relationship with the light-receiving portion 11 that is a function portion pattern of the image pickup chip 10. Hereunder, the alignment marks 31A and 31B are each referred to as alignment mark 31.

Therefore, alignment of the image pickup chip 10 and the cover glass 30 in the image pickup apparatus 1 can be performed accurately and easily. Consequently, manufacture of the image pickup apparatus 1 is easy.

For example, while accuracy when alignment is performed using an outer peripheral portion of the image pickup chip 10 and an outer peripheral portion of the cover glass 30 is around ±20 µm, accuracy in the case of performing alignment using the light-receiving portion 11 and the alignment mark set 31S is ±3 µm or less.

Since the positioning accuracy is high in the structure of the image pickup apparatus 1, there is no risk of reflected light arriving at the light-receiving portion and generating an optical flare due to a side face of the cover glass being too close to the light-receiving portion.

In addition, in the image pickup apparatus 1, the light-receiving portion 11 that is a function portion pattern is used for alignment. Therefore, since it is not necessary to form a mark (alignment mark) that is used only for the purpose of alignment on the image pickup chip 10, miniaturization of the image pickup apparatus 1 is facilitated.

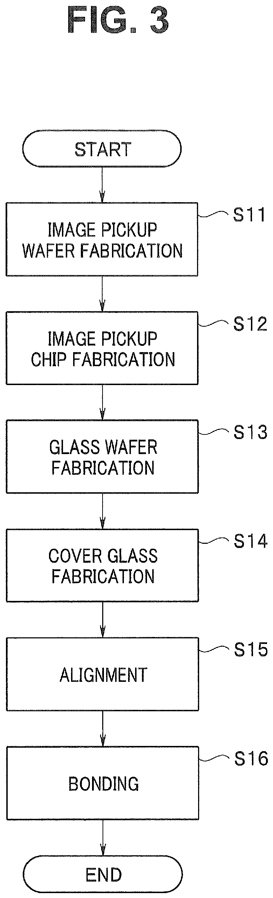

Next, a method for manufacturing the image pickup apparatus 1 will be described with reference to the flowchart in Fig. 3.

### <Step S11> Image Pickup Wafer Fabrication

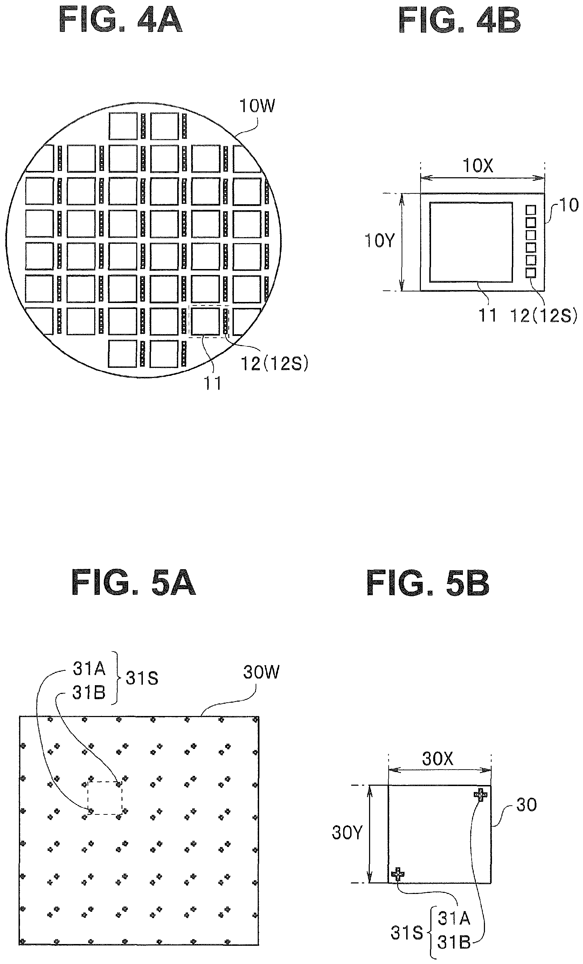

As shown in Fig. 4A, an image pickup wafer 10W on which a plurality of image pickup chip patterns that are made of a plurality of function portion patterns that each include the light-receiving portion 11 and the electrode group 12S are formed is fabricated using known semiconductor manufacturing technology on the main surface of a wafer made of a semiconducting material such as silicon. The light-receiving portion 11 may also be a CCD or the like. Further, a semiconductor circuit that performs primary processing of an image pickup signal may be formed around the light-receiving portion 11. In this case, a semiconductor circuit pattern also constitutes one of the function portion patterns.

### <Step S12> Image Pickup Chip Fabrication

As shown in Fig. 4B, a plurality of rectangular parallelepiped image pickup chips 10 are fabricated by cutting the image pickup wafer 10W at intervals of a width 10X and a height 10Y. As described in the foregoing, the light-receiving portion 11 and the electrode group 12S that includes a plurality of electrode patterns 12 that are connected by wiring (not shown) for sending and receiving signals are arranged on each of the image pickup chips 10.

### <Step S13> Glass Wafer Fabrication

As shown in Fig. 5A, a glass wafer 30W that is an optical member wafer is fabricated. A plurality of alignment mark sets 31S, each of which includes the alignment marks 31A and 31B which are formed at respective positions that are in a predetermined positional relationship with two corner portions that face each other of the light-receiving portion 11 that is a function portion pattern of the image pickup chip 10, are formed on the glass wafer 30W.

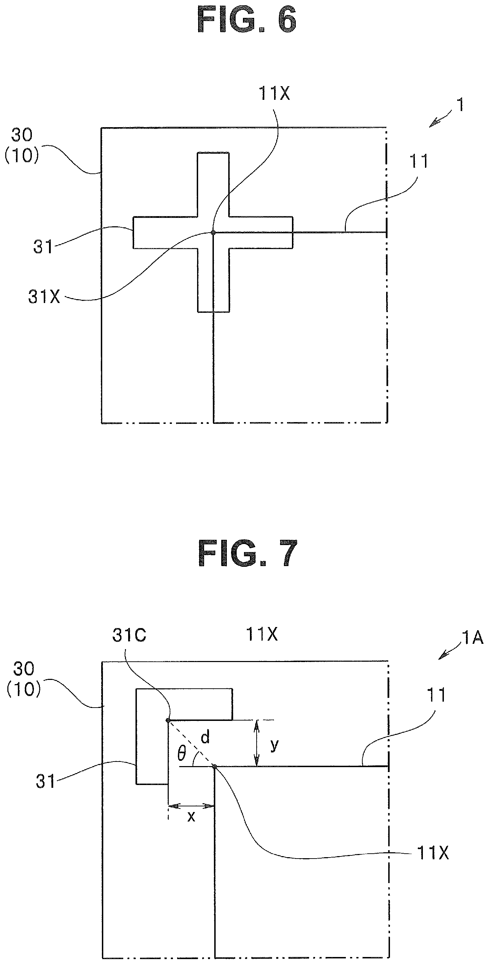

That is, as shown in Fig. 6, a center 31X of the alignment mark 31 that has a cross shape is formed at a position that matches a corner portion 11X of the light-receiving portion 11. In other words, the cover glass 30 is bonded so that the alignment mark 31 covers the corner portion 11X of the light-receiving portion 11. In this connection, with respect to the alignment mark 31A and the alignment mark 31B, because highly accurate alignment is possible when the two alignment marks are separated from each other, the two alignment marks 31A and 31B are formed at positions corresponding to the two corner portions 11X that face each other of the light-receiving portion 11.

The alignment marks 31 are formed of a material that is used for fabrication of the image pickup wafer 10W, such as a metal, silicon nitride, silicon oxide or resin, that is, the alignment marks 31 are formed of a material used in semiconductor manufacturing, and there is no risk of deterioration of the image pickup chip 10 due to contamination.

In particular, it is preferable that the alignment marks 31 be made of a metal such as Cr, Ti or Cu, since it is easy for an image photographed by an alignment apparatus to be formed with a high degree of contrast.

The alignment marks 31, for example, are fabricated by depositing Ti/Cr on one face of a glass wafer made of transparent glass, and thereafter patterning with a photoresist, and then etching.

### <Step S14> Cover Glass Fabrication

As shown in Fig. 5B, a plurality of rectangular parallelepiped cover glasses 30 are fabricated by cutting the glass wafer 30W at intervals in a width direction 30X and a height direction 30Y. In the image pickup apparatus 1, the width 30X of the cover glass 30 is set so that the cover glass 30 covers the light-receiving portion 11 and does not cover the electrode group 12S. That is, the width 30X of the cover glass 30 is less than the width 10X of the image pickup chip. Note that, in a case where alignment accuracy is D µm, the height 30Y of the cover glass 30 is preferably set so as to be reduced by D µm from the height 10Y of the image pickup chip 10.

As described above, on each cover glass 30, the alignment marks 31A and 31B (alignment mark set 31S) are respectively formed at two places that are in a predetermined positional relationship with a function portion pattern of the image pickup chip 10.

In this connection, naturally an order in which the image pickup chip fabrication process and the cover glass fabrication process are performed may be reverse to that described above. Further, as shown in Fig. 4A and Fig. 5A, sizes and shapes of the image pickup wafer 10W and the glass wafer 30W may be different.

### <Step S15> Alignment

An image that is photographed by an alignment apparatus is processed to perform pattern recognition, and alignment of the image pickup chip 10 and the cover glass 30 is performed. That is, by photographing an image of the first main surface 10SA of the image pickup chip 10 and an image of the second main surface 30SB of the cover glass 30 and performing pattern recognition, the corner portion 11X of the light-receiving portion 11 of the image pickup chip 10 and the center 31X of the alignment mark 31 of the cover glass 30 are recognized, and a relative position between the image pickup chip 10 and the cover glass 30 moves to a position at which the corner portion 11X and the center 31X match.

Note that, to prevent a decrease in the alignment accuracy due to a thickness of the cover glass 30, the alignment marks 31 are formed on the second main surface 30SB that is the bonding surface of the cover glass 30.

In addition, since the second main surface 30SB is located in a position at which an image height of the image pickup optical system becomes maximum, adverse effects caused by reflection and scattering of light by the alignment marks 31 are small.

### <Step S16> Bonding

When the image pickup chip 10 and the cover glass 30 are in an aligned state, the image pickup chip 10 and the cover glass 30 are bonded while being pressed together via the adhesive layer 20.

The adhesive layer is selected from among ultraviolet-curing resins or thermosetting resins in consideration of transparency and moisture resistance and the like. The adhesive layer may be a liquid resin or may be a film resin. If the adhesive layer is a film resin, the adhesive layer may be fixed on the cover glass 30 in advance.

In addition, the signal wire 40 is bonded to the electrode pad 12 that is not covered by the cover glass 30.

As described above, according to the method of the present embodiment it is possible to easily manufacture the image pickup apparatus 1 in which the light-receiving portion 11 and the electrode pad 12 are arranged on the first main surface 10SA of the image pickup chip 10, and the light-receiving portion 11 is covered by the cover glass 30.

Further, since the alignment accuracy between the image pickup chip 10 and the cover glass 30 is high, a small cover glass 30 can be used, and thus the image pickup apparatus 1 has a small size.

For example, in a case where the alignment accuracy is 2 µm, in the image pickup apparatus 1, a height of a plan-view dimension thereof is (10Y) or less, and the height will be the same as that of the plan-view dimension of the image pickup chip 10. Further, a width of a plan-view dimension of the image pickup apparatus 1 is also the same as that of the plan-view dimension of the image pickup chip 10.

### <Modifications of First Embodiment>

Next, image pickup apparatuses according to modifications of the first embodiment, and methods for manufacturing the image pickup apparatuses according to the modifications will be described. Since the image pickup apparatuses according to the modifications and the like are similar to the image pickup apparatus 1 of the embodiment, the same constituent elements are assigned the same reference numerals, and a description thereof is omitted.

In manufacturing the image pickup apparatus of the present invention, alignment is performed by processing an image photographed by an alignment apparatus and performing pattern recognition. Therefore, as in the case of the image pickup apparatus 1, it is not necessary for the positions to be overlapping at the time of aligning a part of the alignment mark 31 and a part of the light-receiving portion 11, and it is sufficient that the two components are in a predetermined positional relationship.

Consequently, each of image pickup apparatuses 1A to 1G of the following Modifications 1 to 7 also has the advantageous effects of the image pickup apparatus 1.

### <Modification 1>

In an image pickup apparatus 1A of Modification 1 that is shown in Fig. 7, an inside corner portion 31C of an L-shaped alignment mark 31 is formed so as to be at a predetermined relational position (distance d, relative angle θ) that is separated by distances x and y from the corner portion 11X of the light-receiving portion 11.

For example, highly accurate alignment is enabled by previously inputting the predetermined relational position information (distance d, relative angle θ) into the alignment apparatus.

### <Modification 2>

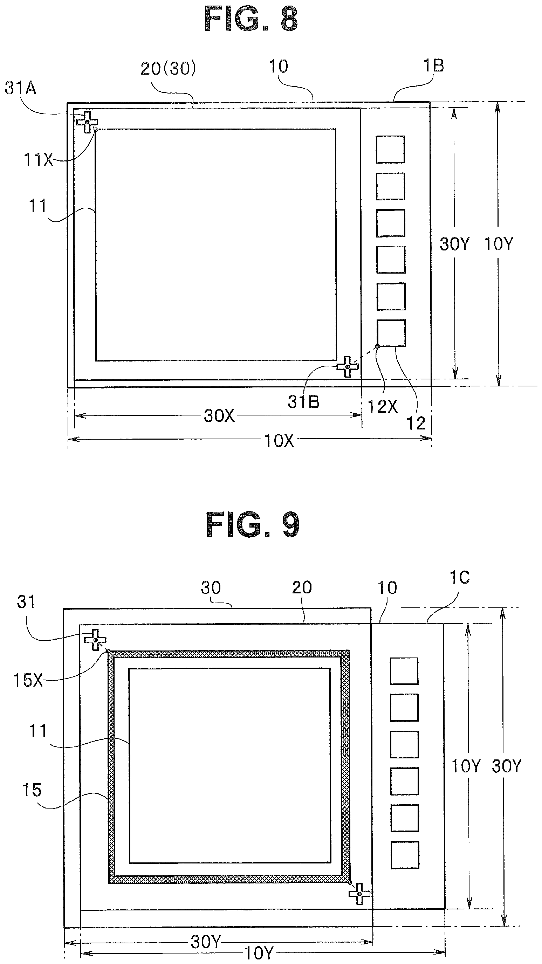

In an image pickup apparatus 1B of Modification 2 that is shown in Fig. 8, similarly to the image pickup apparatus 1A, the alignment mark 31A is in a predetermined positional relationship with the corner portion 11X of the light-receiving portion 11. However, the alignment mark 31B is in a predetermined positional relationship with a corner portion 12X of the electrode pad 12.

That is, the alignment marks 31 may be in a predetermined positional relationship with any of the function portion patterns of the image pickup chip 10.

Further, a cover glass 30B is set so as to be arranged within the surface the image pickup chip 10, and plan-view dimensions of the image pickup apparatus 1A are the same as the plan-view dimensions of the image pickup chip 10.

For example, in a case where the alignment accuracy is ±2 µm, a height of the cover glass 30B is (10Y - 2 µm) or less, and a width is also designed in consideration of the alignment accuracy.

### <Modification 3>

In an image pickup apparatus 1C of Modification 3 that is illustrated in Fig. 9, the alignment mark set 31S is formed at positions that are in a predetermined positional relationship with a guard ring 15 that is a moisture-resistant wall that surrounds the light-receiving portion 11.

The guard ring 15 is a functional pattern that is made of a material having moisture resistance, in particular a material with low moisture permeability such as a metal, and has a function for preventing the infiltration of moisture to the light-receiving portion 11 via the adhesive layer 20.

In this connection, in the image pickup apparatus 1C, a height 30Y of the cover glass 30C is greater than the height 10Y of the image pickup chip 10, and one part of the cover glass 30C also protrudes from a side face of the image pickup chip 10. That is, the dimensions of the cover glass can be set in accordance with the specifications of the image pickup apparatus.

### <Modification 4>

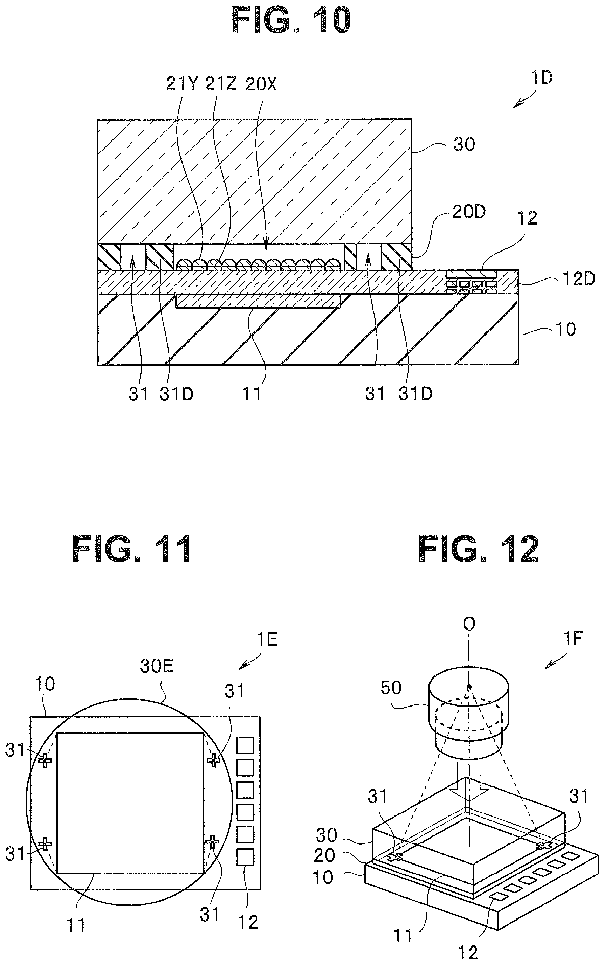

In an image pickup apparatus 1D according to Modification 4 that is illustrated in a cross-sectional schematic diagram in Fig. 10, an adhesive layer 20D does not cover an upper side of the light-receiving portion 11. Note that Fig. 10 is a schematic diagram for describing the image pickup apparatus 1D, which shows sectional views along a plurality of section lines in an appropriately superimposed manner.

A cavity portion (cavity) 20X is formed in the upper side of the light-receiving portion 11 in the image pickup apparatus 1D. To make this kind of shape, for example, the alignment marks 31 that are cavities and the cavity portion 20X in the upper side of the light-receiving portion 11 may be formed by patterning a film-like resin 31D on the surface of the cover glass 30, and the image pickup apparatus 1D may be fabricated so that the remaining resin 31D that is not removed forms the adhesive layer 20D, or the image pickup chip 10 and the cover glass 30 may be bonded via the adhesive layer 20D using an adhesive after forming the cavity portion 20X by using a separate member as a spacer.

The light-receiving portion 11 of the image pickup apparatus 1D is also covered by a transparent insulating layer of a multilayer wiring layer 12D for connecting the light-receiving portion 11 and the electrode pad 12, and a color filter 21Y and a microlens 21Z are further arranged thereon. If the adhesive layer 20D is arranged on the microlens 21Z, a lens function will be lost.

In the image pickup apparatus 1D in which a part of the adhesive layer 20D is the cavity portion 20X, the microlens function will not be lost.

### <Modification 5>

In an image pickup apparatus 1E of Modification 5 that is illustrated in Fig. 11, a cover glass 30E is circular.

In addition, in the cover glass 30E, the alignment marks 31 are respectively formed at four places that are in a predetermined relationship with four corner portions of the light-receiving portion 11, respectively. To achieve highly accurate alignment, it is preferable to form the alignment marks 31 at least at two places, and the alignment marks 31 may be formed at three or more places. In a case where the alignment marks 31 are formed at three or more places, alignment may be performed using two places, and the alignment accuracy may be calculated and corrected using another alignment mark 31.

Note that, as long as an optical member made of a transparent material that covers the light-receiving portion 11 can cover the light-receiving portion 11 and the alignment marks 31 can be formed, the shape thereof may be of a filter such as an infrared cut-off filter or a low-pass filter, a prism, or a member having a lens function or the like.

### <Modification 6>

An image pickup apparatus IF of Modification 6 that is shown in Fig. 12 includes an image pickup optical system 50. In the image pickup apparatus IF, the alignment marks 31 are also utilized for alignment (three axial directions of X, Y and Z) when the image pickup optical system 50 is arranged on the cover glass 30.

### <Modification 7>

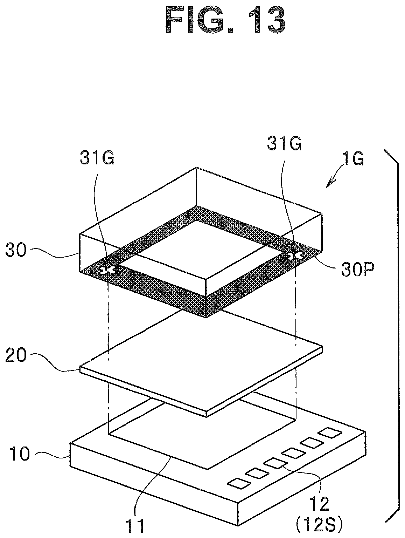

In an image pickup apparatus 1G according to Modification 7 that is shown in Fig. 13, alignment marks 31G are negative patterns that are formed using a light shielding pattern 30P made of metal that prevents light from being incident on the light-receiving portion 11, that is formed at an outer peripheral portion of the main surface (bonding surface) 30SB of the cover glass 30.

The light shielding pattern 30P is a so-called optical mask, and because the alignment marks 31G that are formed utilizing a part thereof can be fabricated simultaneously with formation of the light shielding pattern 30P, it is not necessary to add a new process.

### <Second Embodiment>

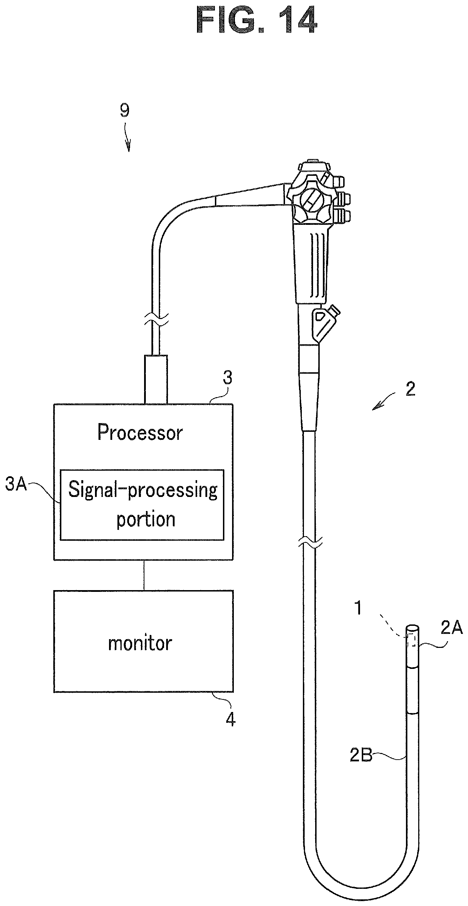

As shown in Fig. 14, an endoscope system 9 according to a second embodiment includes an endoscope 2, a processor 3 and a monitor 4. The image pickup apparatus 1 and the like that are described above are arranged at a distal end portion 2A of an insertion portion 2B of the endoscope 2.

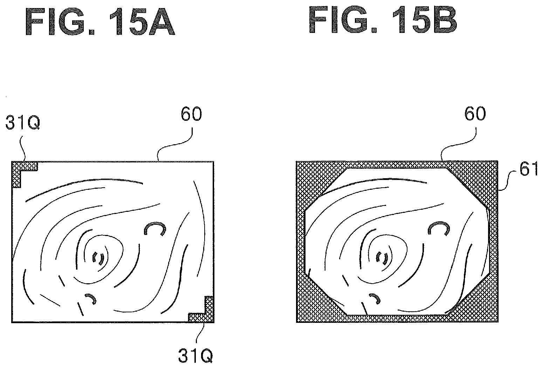

If the alignment marks 31 of the cover glass 30 are bonded so as to cover corner portions of the light-receiving portion 11, as in the configuration of the image pickup apparatus 1 and the like, as shown in Fig. 15A, images 31Q of the alignment marks 31 are displayed in corner portions of a rectangular endoscopic image 60 that the image pickup apparatus 1 picks up.

In the endoscope system 9, because a signal-processing portion 3A of the processor 3 masks the corner portions of an image pickup image by means of an electronic image mask 61, as shown in Fig. 15B, an endoscopic image in which corner portions are not displayed is displayed on the monitor 4.

The endoscope system 9 includes the small-size image pickup apparatus 1 that is easy to manufacture and the like, and furthermore, the unwanted images 31Q of the alignment marks 31 are not displayed in the endoscopic image 60.

The present application claims as a base of priority Japanese Patent Application No. 2013-108138 filed in Japan on May 22, 2013.

## Claims

1. An image pickup apparatus (1), comprising:
a rectangular parallelepiped image pickup chip (10) made of a semiconducting material, in which a plurality of function portion patterns (11, 12) including a light-receiving portion (11) are formed on a first main surface (10SA);
an optical member (30) in which alignment marks (31A, 31B) are formed at at least two places, and which is made of a transparent material that is bonded via an adhesive layer (20) so as to cover the light-receiving portion (11); and
an image pickup optical system arranged in the optical member (30),
**characterized in that** the alignment marks (31A, 31B) are formed on a bonding surface (30SB) of the optical member (30), each of the alignment marks (31A, 31B) is in a predetermined positional relationship with one of the function portion patterns (11, 12) and is formed at a position at which an image height of the image pickup optical system becomes maximum.

2. The image pickup apparatus (1) according to claim 1, wherein:
a plurality of electrode patterns (12) that are connected with the light-receiving portion (11) are provided in a row along an edge on the first main surface (10SA) of the image pickup chip (10); and
the optical member (30) does not cover the plurality of electrode patterns (12).

3. The image pickup apparatus (1) according to claim 1 or 2 wherein the alignment marks (31A, 31B) are formed by a metal layer.

4. The image pickup apparatus (1) according to claim 3, wherein the optical member (30) is a cover glass, a filter (21Y), a prism or a member (21Z) having a lens function.

5. The image pickup apparatus (1) according to claim 4, wherein a plan-view dimension of the image pickup apparatus is identical to a plan-view dimension of the image pickup chip (10).

6. The image pickup apparatus (1) according to claim 4, wherein the alignment marks are formed at positions that are in a predetermined positional relationship with a guard ring that is a moisture-resistant wall that surrounds the light-receiving portion.

7. The image pickup apparatus (1) according to claim 4, wherein the alignment marks (31A, 31B) are formed at positions that are in a predetermined positional relationship with corner portions (11X) of the light-receiving portion (11) that is rectangular in a planar view.

8. The image pickup apparatus (1) according to claim 7, wherein the optical member (30) is bonded so that each of the alignment marks (31A, 31B) covers one of the corner portions (11X) of the light-receiving portion (11).

9. The image pickup apparatus (1) according to any one of claims 1 to 8, wherein the adhesive layer (20) does not cover the light-receiving portion (11).

10. The image pickup apparatus (1) according to any one of claims 1 to 9, wherein the alignment marks (31A, 31B) are formed using a light shielding pattern (30P) that is made of a metal that prevents light from being incident on the light-receiving portion (11) and that is formed at an outer peripheral portion of the bonding surface (30B) of the optical member (30).

11. A method for manufacturing an image pickup apparatus (1), comprising:
a process of fabricating an image pickup chip wafer (10W) made of a semiconducting material in which a plurality of image pickup chip patterns made each of a plurality of function portion patterns (11, 12) that each includes a light-receiving portion (11) are formed on a first main surface (10SA);
a process of fabricating a plurality of rectangular parallelepiped image pickup chips (10) by cutting the image pickup chip wafer (10W) into individual pieces;
a process of forming a plurality of alignment mark sets (31S) on an optical member wafer (30W);
a process of fabricating a plurality of optical members (30) that are rectangular in a planar view and on each of which one of the alignment mark sets (31S) is formed, by cutting the optical member wafer (30W) into individual pieces;
a bonding process of bonding each of the image pickup chips (10) and the optical members (30) via an adhesive layer (20) and
a process of arranging an image pickup optical system in the optical member,
**characterized in that**, in the bonding process, one of the image pickup chips (10) and one of the optical members (30) are bonded while aligning alignment marks (31A, 31B) of the one of the alignment mark sets (31S) and one the function portion patterns (11, 12) that is in a predetermined positional relationship with the alighment marks (31A, 31B), the alignment marks (31A, 31B) being in predetermined positional relationships with at least two places of the function portion patterns (11, 12) and each of the alignment marks (31A, 31B) being formed at a position at which an image of the image pickup optical system becomes maximum.

12. The method for manufacturing an image pickup apparatus (1) according to claim 11, wherein:
in the process of arranging the image pickup optical system, the image pickup optical system is arranged in the optical member (30) utilizing the alignment marks (31A, 31B) for alignment.

13. The method for manufacturing an image pickup apparatus (1) according to claim 11 or 12, wherein:
a plurality of electrode patterns (12) that are connected with the light-receiving portion (11) are provided in a row along an edge on the first main surface (10SA) of the image pickup chip (10); and
the optical member (30) is bonded so as not to cover the plurality of electrode patterns (12).

14. An endoscope system (9), comprising:
an endoscope (2) in which an image pickup apparatus (1) according to any one of claims 1 to 9 is arranged at a distal end portion (2A) of an insertion portion (2B); and
a processor (3) comprising a processing portion (3A) that processes a rectangular endoscopic image that is picked up by the image pickup apparatus (1), masks a corner portion (11X) of the endoscopic image, and outputs an endoscopic image that does not display the corner portion (11X).

## Patentansprüche

1. Bildaufnahmevorrichtung (1) mit:
einem quaderförmigen Bildaufnahmechip (10) aus einem halbleitenden Material, in dem mehrere Funktionsabschnittsmuster (11, 12) mit einem Lichtempfangsabschnitt (11) auf einer ersten Hauptoberfläche (10SA) gebildet sind;
einem optischen Element (30), in dem Ausrichtungsmarkierungen (31A, 31B) an wenigstens zwei Stellen gebildet sind und das aus einem transparenten Material hergestellt ist, das über eine Klebeschicht (20) so gebondet ist, dass der Lichtempfangsabschnitt (11) überdeckt ist; und
einem optischen Bildaufnahmesystem, das in dem optischen Element (30) angeordnet ist,
**dadurch gekennzeichnet, dass** auf einer Bonding-Oberfläche (30SB) des optischen Elements (30) Ausrichtungsmarkierungen (31A, 31B) gebildet sind und sich jede der Ausrichtungsmarkierungen (31A, 31B) in einer vorbestimmten räumlichen Beziehung zu einem der Funktionsabschnittsmuster (11, 12) befindet und an einer Position gebildet ist, an der eine Bildhöhe des optischen Bildaufnahmesystems maximal wird.

2. Bildaufnahmevorrichtung (1) nach Anspruch 1, wobei:
mehrere Elektrodenmuster (12), die mit dem Lichtempfangsabschnitt (11) verbunden sind, in einer Reihe entlang eines Rands der ersten Hauptoberfläche (10SA) des Bildaufnahmechips (10) angeordnet sind; und
das optische Element (30) die mehreren Elektrodenmuster (12) nicht überdeckt.

3. Bildaufnahmevorrichtung (1) nach Anspruch 1 oder 2, wobei die Ausrichtungsmarkierungen (31A, 31B) durch eine Metallschicht gebildet sind.

4. Bildaufnahmevorrichtung (1) nach Anspruch 3, wobei das optische Element (30) ein Abdeckglas, ein Filter (21Y), ein Prisma oder ein Element (21Z) mit einer Linsenfunktion ist.

5. Bildaufnahmevorrichtung (1) nach Anspruch 4, wobei eine Draufsichtsabmessung der Bildaufnahmevorrichtung identisch ist mit einer Draufsichtsabmessung des Bildaufnahmechips (10).

6. Bildaufnahmevorrichtung (1) nach Anspruch 4, wobei die Ausrichtungsmarkierungen an Positionen gebildet sind, die sich in einer vorbestimmten räumlichen Beziehung zu einem Schutzring befinden, der eine feuchtigkeitsbeständige Wand umfasst, die den Lichtempfangsabschnitt umgibt.

7. Bildaufnahmevorrichtung (1) nach Anspruch 4, wobei die Ausrichtungsmarkierungen (31A, 31B) an Positionen gebildet sind, die sich in einer vorbestimmten räumlichen Beziehung zu Eckabschnitten (11X) des Lichtempfangsabschnitts (11), der in einer Draufsicht rechteckig ist, befinden.

8. Bildaufnahmevorrichtung (1) nach Anspruch 7, wobei das optische Element (30) so gebondet ist, dass jede der Ausrichtungsmarkierungen (31A, 31B) einen der Eckabschnitte (11X) des Lichtempfangsabschnitts (11) überdeckt.

9. Bildaufnahmevorrichtung (1) nach einem der Ansprüche 1 bis 8, wobei die Klebeschicht (20) den Lichtempfangsabschnitt (11) nicht überdeckt.

10. Bildaufnahmevorrichtung (1) nach einem der Ansprüche 1 bis 9, wobei die Ausrichtungsmarkierungen (31A, 31B) unter Verwendung eines Lichtabschirmungsmusters (30P) gebildet sind, das aus einem Material hergestellt ist, das verhindert, dass Licht auf den Lichtempfangsabschnitt (11) fällt, und das an einem Außenumfangsabschnitt der Bonding-Oberfläche (30B) des optischen Elements (30) gebildet ist.

11. Verfahren zur Herstellung einer Bildaufnahmevorrichtung (1) mit:
einem Prozess zum Herstellen eines Bildaufnahmechipwafers (10W) aus einem halbleitenden Material, in dem mehrere Bildaufnahmechipmuster, die jeweils aus mehreren Funktionsabschnittsmuster (11, 12) gebildet sind, die jeweils einen Lichtempfangsabschnitt (11) umfassen, auf einer ersten Hauptoberfläche (10SA) gebildet sind;
einem Prozess zum Herstellen mehrerer quaderförmiger Bildaufnahmechips (10) durch Schneiden des Bildaufnahmechipwafers (10W) in einzelne Teile;
einem Prozess zum Bilden mehrerer Ausrichtungsmarkierungssätze (31S) auf einem Wafer (30W) mit optischem Element;
einem Prozess zum Herstellen mehrerer optischer Elemente (30), die in einer Draufsicht rechteckig sind und wobei auf jedem von ihnen einer der Ausrichtungsmarkierungssätze (31S) gebildet ist, durch Schneiden des Wafers (30W) mit optischem Element in einzelne Teile;
einem Bondingprozess zum Bonden von jedem der Bildaufnahmechips (10) und den optischen Elementen (30) über eine Klebeschicht (20) und
einem Prozess zum Anordnen eines optischen Bildaufnahmesystems in dem optischen Element,
**dadurch gekennzeichnet, dass** in dem Bondingprozess einer der Bildaufnahmechips (10) und eines der optischen Elemente (30) gebondet werden, während Ausrichtungsmarkierungen (31A, 31B) von dem einen der Ausrichtungsmarkierungssätze (31S) und eines der Funktionsabschnittsmuster (11, 12), das sich in einer vorbestimmten räumlichen Beziehung mit den Ausrichtungsmarkierungen (31A, 31B) befindet, gebondet werden, wobei sich die Ausrichtungsmarkierungen (31A, 31B) in vorbestimmten räumlichen Beziehungen zu wenigstens zwei Stellen der Funktionsabschnittsmuster (11, 12) befinden und jede der Ausrichtungsmarkierungen (31A, 31B) an einer Position gebildet ist, an der ein Bild des optischen Bildaufnahmesystems maximal wird.

12. Verfahren zur Herstellung einer Bildaufnahmevorrichtung (1) nach Anspruch 11, wobei:
in dem Prozess zum Anordnen des optischen Bildaufnahmesystems das optische Bildaufnahmesystem in dem optischen Element (30), das die Ausrichtungsmarkierungen (31A, 31B) zur Ausrichtung nutzt, angeordnet wird.

13. Verfahren zur Herstellung einer Bildaufnahmevorrichtung (1) nach Anspruch 11 oder 12, wobei:
mehrere Elektrodenmuster (12), die mit dem Lichtempfangsabschnitt (11) verbunden sind, in einer Reihe entlang eines Rands der ersten Hauptoberfläche (10SA) des Bildaufnahmechips (10) angeordnet werden; und
das optische Element (30) so gebondet wird, dass die mehreren Elektrodenmuster (12) abgedeckt sind.

14. Endoskopsystem (9) mit:
einem Endoskop (2), in dem eine Bildaufnahmevorrichtung (1) nach einem der Ansprüche 1 bis 9 an einem distalen Endabschnitt (2A) eines Einführungsabschnitts (2B) angeordnet ist; und
einem Prozessor (3), der einen Verarbeitungsabschnitt (3A), der ein rechteckiges Endoskopiebild, das durch die Bildaufnahmevorrichtung (1) aufgenommen wird, umfasst, einen Eckabschnitt (11X) des Endoskopiebilds maskiert und ein Endoskopiebild, das den Eckabschnitt (11X) nicht zeigt, ausgibt.

## Revendications

1. Appareil de prise d'images (1), comprenant :
une puce de prise d'images parallélépipédique rectangulaire (10) réalisée en matériau semi-conducteur, dans laquelle une pluralité de motifs de portions fonctionnelles (11, 12) incluant une portion de réception de lumière (11) sont formés sur une première surface principale (10SA) ;
un élément optique (30) dans lequel des marques d'alignement (31A, 31B) sont formées à au moins deux emplacements, et qui est fait d'un matériau transparent qui est collé via une couche adhésive (20) de manière à couvrir la portion de réception de lumière (11); et
un système optique de prise d'images agencé dans l'élément optique (30),
**caractérisé en ce que** les marques d'alignement (31A, 31B) sont formées sur une surface de collage (30SB) de l'élément optique (30), chacune des marques d'alignement (31A, 31B) étant dans une relation positionnelle prédéterminée avec l'un des motifs de portions fonctionnelles (11, 12) et est formé à une position à laquelle la hauteur d'image du système optique de prise d'images devient maximum.

2. Appareil de prise d'images (1) selon la revendication 1), dans lequel :
une pluralité de motifs d'électrodes (12) qui sont connectés avec la portion de réception de lumière (11) sont prévus dans une rangée le long d'une bordure sur la première surface principale (10SA) de la puce de prise d'images (10) ; et
l'élément optique (30) ne couvre pas la pluralité de motifs d'électrodes (12).

3. Appareil de prise d'images (1) selon la revendication 1 ou 2, dans lequel :
les marques d'alignement (31A, 31B) sont formées par une couche de métal.

4. Appareil de prise d'images (1) selon la revendication 3, dans lequel l'élément optique (30) est un verre de couverture, un filtre (21Y), un prisme ou un élément (21Z) ayant la fonction d'une lentille.

5. Appareil de prise d'images (1) selon la revendication 4, dans lequel une dimension, vue dans un plan, de l'appareil de prise d'images est identique à une dimension, vue dans un plan, de la puce de prise d'images (10).

6. Appareil de prise d'images (1) selon la revendication 4, dans lequel les marques d'alignement sont formées à des positions qui sont dans une relation positionnelle prédéterminée avec un anneau de garde qui est une paroi résistante à l'humidité qui entoure la portion de réception de lumière.

7. Appareil de prise d'images (1) selon la revendication 4, dans lequel les marques d'alignement (31A, 31B) sont formées à des positions qui sont dans une relation positionnelle prédéterminée avec des portions de coin (11X) de la portion de réception de lumière (11) qui est rectangulaire dans une vue en plan.

8. Appareil de prise d'images (1) selon la revendication 7, dans lequel l'élément optique (30) est collé de telle façon que chacune des marques d'alignement (31A, 31B) couvre l'une des portions de coin (11X) de la portion de réception de lumière (11).

9. Appareil de prise d'images (1) selon l'une quelconque des revendications 1 à 8, dans lequel la couche adhésive (20) ne couvre pas la portion de réception de lumière (11).

10. Appareil de prise d'images (1) selon l'une quelconque des revendications 1 à 9, dans lequel les marques d'alignement (31A, 31B) sont formées en utilisant un motif formant écran à la lumière (30P) qui est fait d'un métal qui empêche à la lumière de tomber sur la portion de réception de lumière (11) et qui est formé à une portion périphérique extérieure de la surface de collage (30B) de l'élément optique (30).

11. Procédé pour fabriquer un appareil de prise d'images (1), comprenant :
une procédure consistant à fabriquer une plaquette de puces de prise d'images (10W) faite d'un matériau semi-conducteur dans laquelle une pluralité de motifs de puces de prise d'images, constitué chacun d'une pluralité de motifs de portions fonctionnelles (11, 12) qui incluent chacun une portion de réception de lumière (11), sont formés sur une première surface principale (10SA);
une procédure consistant à fabriquer une pluralité de puces de prise d'images parallélépipédique rectangulaire (10) en coupant la plaquette de puces de prise d'images (10W) en pièces individuelles ;
un processus consistant à former une pluralité de groupes de marques d'alignement (31S) sur une plaquette d'éléments optiques (30W) ;
une procédure consistant à fabriquer une pluralité d'éléments optiques (30) qui sont rectangulaires dans une vue en plan et sur chacun desquels est formé l'un des groupes de marques d'alignement (31S), en coupant la plaquette d'éléments optiques (30W) en pièces individuelles;
une procédure de collage consistant à coller chacune des puces de prise d'images (10) et des éléments optiques (30) via une couche adhésive (20) ; et
une procédure consistant à agencer un système optique de prise d'images dans l'élément optique,
**caractérisé en ce que**, dans la procédure de collage, l'une des puces de prise d'images (10) et l'un des éléments optiques (30) sont collés tout en alignant des marques d'alignement (31A, 31B) de l'un des groupes de marques d'alignement (31S) et l'un des motifs de portions fonctionnelles (11, 12) qui est dans une relation positionnelle prédéterminée avec les marques d'alignement (31A, 31B), les marques d'alignement (31A, 31B) étant dans des relations positionnelles prédéterminées avec au moins deux emplacements des motifs de portions fonctionnelles (11, 12) et chacune des marques d'alignement (31A, 31B) étant formée à une position à laquelle une image du système optique de prise d'images devient maximum.

12. Procédé pour fabriquer un appareil de prise d'images (1) selon la revendication 11, dans lequel :
dans la procédure d'agencement du système optique de prise d'images, le système optique de prise d'images est agencé dans l'élément optique (30) en utilisant les marques d'alignement (31A, 31B) pour l'alignement.

13. Procédé pour fabriquer un appareil de prise d'images (1) selon la revendication 11 ou 12, dans lequel :
une pluralité de motifs d'électrodes (12) qui sont connectés avec la portion de réception de lumière (11) sont prévus dans une rangée le long d'une bordure sur la première surface principale (10SA) de la puce de prise d'images (10) ; et
l'élément optique (30) est collé de manière à ne pas couvrir la pluralité de motifs d'électrodes (12).

14. Système à endoscope (9), comprenant :
un endoscope (2) dans lequel un appareil de prise d'images (1) selon l'une quelconque des revendications 1 à 9 est agencé à une portion d'extrémité distale (2A) d'une portion d'insertion (2B) ; et
un processeur (3) comprenant une portion de traitement (3A) qui traite une image endoscopique rectangulaire qui est prise par l'appareil de prise d'images (1), qui masque une portion de coin (11X) de l'image endoscopique, et qui délivre une image endoscopique qui n'affiche pas la portion de coin (11X).
